# EUROPEAN PATENT SPECIFICATION

(11) **EP 3 206 487 B1**
(45) Date of publication and mention of the grant of the patent: **29.12.2021**
(21) Application number: 15781614.1
(22) Date of filing: 08.10.2015
(51) Int. Cl.: A01N 31/02, A01N 37/02, A01N 25/30, A01P 1/00, A61P 31/02, A61P 31/12

(54) **USE OF FATTY ACID ESTER FOR IMPROVING THE ANTIMICROBIAL EFFICACY OF AN ALCOHOLIC DISINFECTANT**
VERWENDUNG EINES FETTSÄUREESTERS ZUR VERBESSERUNG DER ANTIMIKROBIELLEN WIRKSAMKEIT EINES ALKOHOLISCHEN DESINFEKTIONSMITTELS
UTILISATION D'ESTER D'ACIDE GRAS POUR AMÉLIORER L'EFFICACITÉ ANTIMICROBIENNE D'UN DÉSINFECTANT À BASE D'ALCOOL

(30) Priority: 16.10.2014 DE 102014115080
(43) Date of publication of application: 23.08.2017
(73) Proprietor: Schülke & Mayr GmbH, 22851 Norderstedt (DE)
(72) Inventor: DA SILVA NOLASCO, Angelo, 20537 Hamburg (DE); KOLDITZ, Petra, 22145 Hamburg (DE); STEINHAUER, Katrin, 22459 Hamburg (DE); BOCK, Sylvia, 24882 Schaalby (DE)
(74) Representative: LKGLOBAL Lorenz & Kopf PartG mbB Patentanwälte
(86) International application number: PCT/EP2015/073279
(87) International publication number: WO 2016/058902

(56) References cited:
- EP-A1- 1 683 416
- EP-A1- 2 462 806
- EP-A2- 0 375 827
- WO-A1-2008/034224
- WO-A1-2010/002880
- WO-A2-2004/062589
- WO-A2-2009/088894
- WO-A2-2012/084164
- US-A1- 2001 033 854
- US-A1- 2003 139 307
- US-A1- 2012 208 894
- US-A1- 2012 214 878

## Description

The invention relates to an alcoholic disinfectant and the disinfectant for use for hygienic and for surgical disinfection of the hands. The invention further relates to the use of fatty acid ester for improving the antimicrobial efficacy of an alcoholic disinfectant.

For hand hygiene and hand disinfection, for example ethanol-based disinfectants are recommended (including the product desderman^{®} pure from Schülke & Mayr GmbH with a content (according to manufacturer's data) of 78.2 g ethanol (96%) and 0.1 g biphenyl-2-ol in 100 g). However, there are misgivings regarding disinfectants for the hygienic and surgical disinfection of the hands that contain high proportions of ethanol, because agents of this kind have a very strong defatting action.

When using ethanolic hand disinfectants, refatting of the skin thus plays an important role for user compliance. Glycerin is described in the literature as having skin-care qualities (Atrux-Tallau, N., Romagny, C., Padois, K., Denis, A., Haftek, M., Falson, F., Pirot, F., and Maibach, H. I. (2010). Arch Dermatol Res 302: 435-441). Glycerin is therefore a widely used substance as refatting agent in commercial formulations for alcoholic disinfection of the hands (cf. for example the commercial products Sterillium^{®} classic and Sterillium^{®} med from Bode Chemie GmbH, Hamburg, Federal Republic of Germany).

WO2014/032696 A1 discloses virucidal alcoholic hand disinfectants. In addition to C₂ to C₃ alcohol, the presence of glycerin is mandatory. However, agents with virucidal action are not necessarily effective against all microorganisms against which hand disinfectants must be effective. Moreover, investigations with glycerin-containing formulations (WHO-I and WHO-II) have shown that the presence of glycerin inhibits the bactericidal properties of alcoholic hand disinfectants for surgical disinfection of the hands according to EN 12791 (Suchomel, M., Rotter, M. Weinlich, M., Kundi, M. (2013). J Hosp Infect 83: 284-7).

WO2006/122345 A1 describes agents for treating areas of skin before surgery. However, the glycerin-free gel as humectant, disclosed for example in WO 2006/122345 A1, contains relatively little ethanol (70 wt%). Moreover, it contains triclosan, which is assessed critically by the Federal Institute for Risk Assessment of the Federal Republic of Germany (FRG), cf. Opinion No. 30/2006 dated 8 May 2006, namely because bacteria such as *Salmonella enterica, Escherichia coli, Pseudomonas aeruginosa* and *Staphylococcus aureus* have mechanisms for developing resistance to triclosan.

WO 2009/088894 A2 discloses skin disinfectants containing C₂ to C₅ alcohol. They may also contain humectant esters. For example, the teaching includes esters of alkane diacids, especially the diisopropyl esters of adipic acid (hexane diacid) and sebacic acid (decane diacid). However, the efficacy of a disinfectant largely depends on the alcohol used and its concentration in the disinfectant (or the mixture of alcohols used and the concentration thereof).

EP 0 375 827 A2 relates to a disinfectant composition for medical use comprising ethanol used for disinfection, a bactericidal agent, and an emollient, wherein said bactericidal agent is at least one selected from the group consisting of chlorhexidine digluconate, alkylpyridinium salts, and quaternary ammonium salts, and said emollient is at least one selected from the group consisting of diesters of dibasic acid, triesters of citric acid, triesters of phosphoric acid, polyoxyethylene glyceride of higher fatty acid, and polyglycerol.

EP 2 462 806 A1 relates to the use of aliphatic alcohols in the production of a low-spore or spore-free disinfectant for preventing contamination of the disinfectant with spores and to a process for producing a low-spore or spore-free disinfectant, in which aliphatic alcohol is added to reduce or prevent contamination with spores.

EP 1 683 416 A1 relates to alcoholic compositions which include a) one or more 1- or 2- (C₁ - to C₂₄ -alkyl)-glycerol ethers, b) one or more bispyridiniumalkanes and c) one or more aliphatic alcohols, and to the use of the compositions for the disinfection of animate and inanimate surfaces, in particular for surgical and hygienic hand disinfection and disinfectant handwashing.

US 2003/0139307 A1 relates to a liquid cleansing product that effectively reduces the level of microbes on the skin in a relatively short wash time and which dries quickly without causing damage and drying to the skin. The sanitizing cleanser composition comprising an effective amount of alcohol to produce a reduction in microorganisms on the surface of the skin, and an additive to maintain the skin pH in the range of from about 4.0 to about 6.0.

The present invention is based on the problem of supplying refatting agents for ethanolic disinfectants, wherein the refatting agents in the disinfectant should not, in contrast to glycerin, have an inhibitory effect on the antimicrobial efficacy, especially when they are to be used at higher concentration.

It was found that this problem is solved according to the invention by using isopropyl myristate. Thus, according to the invention it was found, surprisingly, that addition of isopropyl myristate as refatting agent to an alcoholic disinfectant leads, with increasing concentration, even to an improvement in efficacy in the quantitative suspension test according to DGHM, in contrast to addition of glycerin as refatting agent.

The invention therefore relates to an alcoholic disinfectant that comprises
a) 81.5 to 92.5 wt% ethanol, relative to the weight of the disinfectant, and
b) 0.3 to 2.0 wt% of isopropyl myristate,
with the disinfectant comprising
i. less than 0.1 wt% glycerin, relative to the weight of the disinfectant, and
ii. less than 5.0 wt% perfume, relative to the weight of the disinfectant,
the disinfectant comprising 0.05 to 5.0 wt% ingredients other than water and ingredients a) and b).

The disinfectant according to the invention thus comprises less than 0.1 wt% glycerin, preferably less than 0.05 wt%, especially less than 0.03 wt%, for example less than 0.01 wt% glycerin, in each case relative to the weight of the disinfectant.

### a) Ethanol

The alcoholic disinfectant according to the invention contains 81.5 to 92.5 wt% ethanol, in each case relative to the weight of the disinfectant.

The stated ethanol content thus refers to the pure substance (and does not include denaturing agent or water contained for example in ethanol of technical or medical purity).

### b) Isopropyl myristate

Disinfectants according to the invention contain b) isopropyl myristate.

Component b), isopropyl myristate, is present in the disinfectant according to the invention in an amount from 0.3 to 2.0 wt%, such as 0.5 to 1.8 wt%, for example 0.6 to 1.6 wt%, such as 0.75 or about 1.4 wt%.

### Further constituents of the disinfectant

In addition to the mandatory components a) and b), disinfectants according to the invention may further optionally contain:

### c) Fatty alcohol

The fatty alcohol is preferably selected from C₁₀ to C₁₈ fatty alcohols, preferably C₁₂ to C₁₆ fatty alcohols, wherein the fatty alcohol is preferably myristyl alcohol.

Component c), preferably myristyl alcohol, is present in the disinfectant according to the invention preferably in an amount from 0.02 to 5 wt%, relative to the weight of the disinfectant, more preferably 0.1 to 4 wt%, for example 0.5 to 1.5 wt%, such as 1.0 wt%.

### d) Diol

Preferred diols contained in the disinfectant according to the invention are propanediol-1,2 and propanediol-1,3.

Component d) is present in the disinfectant according to the invention preferably in an amount from 0.01 to 2 wt%, relative to the weight of the disinfectant, more preferably 0.1 to 2 wt%, especially 0.2 to 1.5 wt%, such as 0.4 to 1.0 wt%, for example about 0.5 or about 0.8 wt%.

### e) Bispyridinium alkane

Here, the term bispyridinium alkane comprises the bis-[4-(substituted-amino)-1-pyridinium]-alkanes of general formulae (I) or (II) disclosed in DE 27 08 331 C2 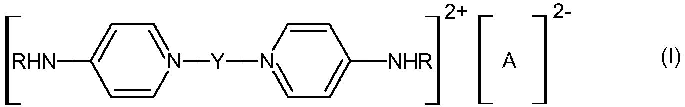 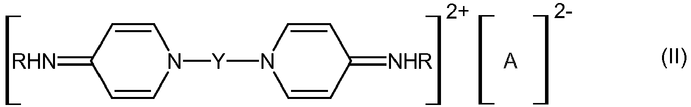 in which
Y is an alkylene group with 4 to 18 carbon atoms,
R denotes an alkyl group with 6 to 18 carbon atoms or a cycloalkyl group with 5 to 7 carbon atoms or the phenyl residue, which is substituted with a halogen atom, and
A is an anion or several anions.

The above definition for A applies strictly speaking to mono- and divalent anions, but A may of course also be a polyvalent anion, e.g. phosphate or orthosilicate. Furthermore, the term bispyridinium alkane comprises the various prototropes of the compounds of formula (I), as is disclosed for example in DE 196 47 692 A1.

In all embodiments of the invention it is preferred that the bispyridinium alkane is octenidine dihydrochloride (R = n-octyl, Y = n-decenyl; A = 2 x Cl, "octenidine") . Component e) is thus especially preferably octenidine.

Preferred amounts of component e) in the disinfectant according to the invention are 0.005 to 1.0 wt%, preferably 0.01 to 0.5 wt%, more preferably 0.03 to 0.3 wt%, even more preferably 0.04 to 0.2 wt%, such as 0.05 to 0.15 wt%, for example about 0.1 wt%, in each case relative to the total weight of the disinfectant.

### f) Chlorhexidine derivative

An example of a chlorhexidine derivative is chlorhexidine gluconate.

Component f) is present in the disinfectant according to the invention preferably in an amount from 0.01 to 2 wt%, relative to the weight of the disinfectant, more preferably 0.1 to 2 wt%, especially 0.2 to 1.5 wt%, such as 0.3 to 1.0 wt%, for example about 0.4 wt%.

### g) Glycerin ether

An especially preferred glycerin ether is 1-(2-ethylhexyl)glycerin ether (Sensiva^{®} SC 50).

The amount of glycerin ether (especially 1-(2-ethylhexyl)glycerin ether) is preferably 0.01 to 1 wt%, relative to the weight of the disinfectant, preferably 0.02 to 0.5 wt%, such as 0.05 to 0.3 wt%, for example about 0.2 wt%.

### h) Fatty acid fatty alcohol ester

Disinfectants according to the invention preferably contain h) fatty acid fatty alcohol esters.

The fatty alcohol of the fatty acid fatty alcohol ester is preferably selected from C₁₀ to C₁₈ fatty acids, preferably C₁₂ to C₁₆ fatty acids, and is for example cetearyl alcohol.

The fatty acid of the fatty acid fatty alcohol ester is preferably selected from short-chain fatty acids, such as C₄ to C₁₂ fatty acids, especially C₈ to C₁₀ fatty acids, wherein the fatty acid is preferably octanoic acid.

Therefore in all embodiments of the present invention it is preferred that component h) is cetearyl octanoate.

Component h), preferably cetearyl octanoate, is present in the disinfectant according to the invention preferably in an amount from 0.01 to 2 wt%, relative to the weight of the disinfectant, more preferably 0.1 to 2 wt%, especially 0.2 to 1.5 wt%, such as 0.3 to 1.0 wt%, for example about 0.8 wt%.

### i) Polyvinylpyrrolidone

An excipient preferably contained in the disinfectant according to the invention is polyvinylpyrrolidone, preferably povidone K, especially povidone K30.

Component i), i.e. polyvinylpyrrolidone, is present in the disinfectant according to the invention preferably in an amount from 0.01 to 2 wt%, relative to the weight of the disinfectant, more preferably 0.1 to 2 wt%, especially 0.2 to 1.5 wt%, such as 0.4 to 1.0 wt%, for example about 0.3 wt%.

### j) Sugar alcohol

In an especially preferred embodiment, the disinfectant according to the invention contains j) sugar alcohol, for example sorbitol.

Component j) is present in the disinfectant according to the invention preferably in an amount from 0.01 to 2 wt%, relative to the weight of the disinfectant, more preferably 0.1 to 2 wt%, especially 0.2 to 1.5 wt%, such as 0.3 to 1.0 wt%, for example about 0.5 wt%.

### k) Antioxidant

Preferably, disinfectants according to the invention contain k) one or more antioxidants. Examples of antioxidants are BHA, BHT or vitamin E (or derivatives thereof), especially preferably vitamin E (or vitamin E-acetate).

The amount of antioxidant (especially tocopherol and/or tocopherol acetate) is preferably 0.001 to 0.1 wt%, relative to the weight of the disinfectant, preferably 0.002 to 0.05 wt%, such as 0.005 to 0.03 wt%, for example about 0.01 wt%.

### c) Skin care agent

Skin care agents 1) optionally present in the disinfectant according to the invention are preferably urea, dexpanthenol, hamamelis, bisabolol and/or allantoin.

The amount of component 1) (especially dexpanthenol) is preferably 0.01 to 1 wt%, relative to the weight of the disinfectant, preferably 0.02 to 0.5 wt%, such as 0.05 to 0.3 wt%, for example about 0.1 or 0.2 wt%.

The disinfectant contains, apart from the mandatory components a) and b), only 0.05 to 5.0 wt%, preferably 1.0 to 4.0 wt% of further ingredients and water as the remainder.

Moreover, it is preferred that the disinfectant comprises less than 1.0 wt% of water-insoluble polymeric amines (as are described as mandatory component in DE 10 2009 040 089 A1 relating to deodorants). Preferably the disinfectant contains less than 0.5 wt% of water-insoluble polymeric amines, especially less than 0.2 wt%, such as for example less than 0.1 wt%, in each case relative to the weight of the disinfectant, wherein especially preferably the disinfectant is free from water-insoluble polymeric amines.

In particular, disinfectants according to the invention are preferred that comprise less than 1.0 wt% of amino-substituted polystyrenes (as are described as preferred water-insoluble polymeric amines in DE 10 2009 040 089 A1 relating to deodorants). Preferably the disinfectant contains less than 0.5 wt% of amino-substituted polystyrenes, especially less than 0.2 wt%, for example less than 0.1 wt%, in each case relative to the weight of the disinfectant, wherein especially preferably the disinfectant is free from amino-substituted polystyrenes.

In all embodiments of the present invention, it is preferred that the disinfectant comprises less than 5.0 wt% DMSO, for example less than 3.0 wt%, in each case relative to the weight of the disinfectant. Especially preferably, the disinfectant according to the invention contains less than 1.0 wt%, especially less than 0.5 wt%, for example less than 0.1 wt% DMSO, in each case relative to the weight of the disinfectant, wherein an especially preferred disinfectant according to the invention is free from DMSO.

Disinfectants according to the invention are preferably in the form of solution.

Alternatively, the disinfectants according to the invention may be in the form of aqueous-alcoholic gels (hydrogels). Hydrogels are prized owing to their transparency and their non-greasy character. Lipophilic gels (oleogels) are also used owing to their aesthetic form and their consistency-imparting properties. Gels are mainly intended for external use and should be applied thinly.

A hydrogel is a generally translucent mass, which is produced using gelatin, tragacanth, Carbopol or similar swelling substances with addition of water.

In the embodiment according to the invention in which the disinfectant is in the form of gel, it preferably contains
a) 81,5 to 85 wt% ethanol,
b) 0.5 to 1.5 wt% isopropyl myristate,
c) 0.8 to 1.5 wt% fatty alcohol (preferably myristyl alcohol),
m) 0.1 to 0.6 wt% thickener (preferably polyacrylic acid copolymer) and
n) 0.3 to 1.0 wt% complexing agent (preferably N,N,N',N'-tetrakis-(2-hydroxypropyl)-ethylenediamine).

Preferred gels according to the invention further contain
d) 0.2 to 3.0 wt% diol (preferably propylene glycol),
j) 0.2 to 0.5 wt% sugar alcohol (preferably sorbitol),
k) 0.005 to 0.2 wt% of antioxidant (preferably tocopherol and/or tocopherol acetate),
1) 0.01 to 0.1 wt% of skin care agent (preferably bisabolol, hamamelis and/or dexpanthenol), and
o) 0.02 to 0.05 wt% of UV-stabilizer.

Further described is an alcoholic disinfectant as described above for use for surgical and hygienic disinfection of the hands.

Further described is the use of isopropyl myristate for improving the antimicrobial efficacy of an alcoholic disinfectant, wherein the disinfectant is as described above. Preferably this improvement in antimicrobial efficacy is an improvement in the quantitative suspension test according to DGHM (Method 9, Gebel et al. (2001): Standard methods of the DGHM for testing chemical methods of disinfection, mhp Verlag GmbH, as at 1 September 2001), determined at a concentration of 35% (v/v) of the disinfectant in the test (diluted with water of standardized hardness (WSH) on a concentration of 43.75% (v/v) (i.e. 1.25-fold) dirty conditions, *Escherichia coli* K12 ATCC 10538), with time of action of 30 s, 60 s, 90 s or 120 s (and preferably 60 s) .

The advantages of the present invention can be seen in particular from the following examples. Unless stated otherwise, all percentages refer to weight.

### Examples

Abbreviations used:
IPM = isopropyl myristate
EtOH = ethanol

### I. Methods:

### Method A - Antimicrobial efficacy

Quantitative suspension test according to DGHM, determined at a final concentration in the test of 35% (v/v) of the disinfectant (Gebel et al. (2001): Standard methods of the DGHM for testing chemical methods of disinfection, mhp Verlag GmbH, as at 1 September 2001). The individual test samples were prepared at a concentration of 43.75% (v/v) (i.e. 1.25-fold higher in water of standardized hardness (WSH, as described in Gebel et al. (2001): Standard methods of the DGHM for testing chemical methods of disinfection, mhp Verlag GmbH, as at 1 September 2001), diluted in order to reach the final concentration required in the test of 35% (v/v)) of the disinfectant, to allow testing in the dynamic range. *Escherichia coli* K12 ATCC 10538 was used as test organism. The tests were carried out in conditions of high organic loading (dirty conditions: 0.3% (g/100 ml) bovine serum albumin (fraction V) + 0.3% (g/100 ml) sheep erythrocytes) according to Method 9 (quantitative suspension test) Standard methods of the DGHM for testing chemical disinfectants (Gebel et al. (2001) with the stated test intervals (30, 60, 90, 120 seconds).

Evaluation:
The tests were evaluated according to Method 9, Quantitative suspension test, Standard methods of the DGHM for testing chemical disinfectants (Gebel et al. (2001)). The microbiological efficacy is stated as log RF (logarithmic reduction factor), which is calculated as follows: log RF = log (CFU Co1) - log (CFU D), with CFU Co1: number of colony forming units (CFU) per ml without action of the preparation (WSH-control (WSH = water of standardized hardness)) and CFU D: number of CFUs per ml after action of the preparation.

### Method B - Haptic assessment

The proband test was conducted with blinded samples over a period of 6 weeks. Assessment by the probands (in each case 10) was based on the school grading system from 1 to 6.

### Example 1

### Antimicrobial efficacy of an ethanolic solution with addition of isopropyl myristate or glycerin

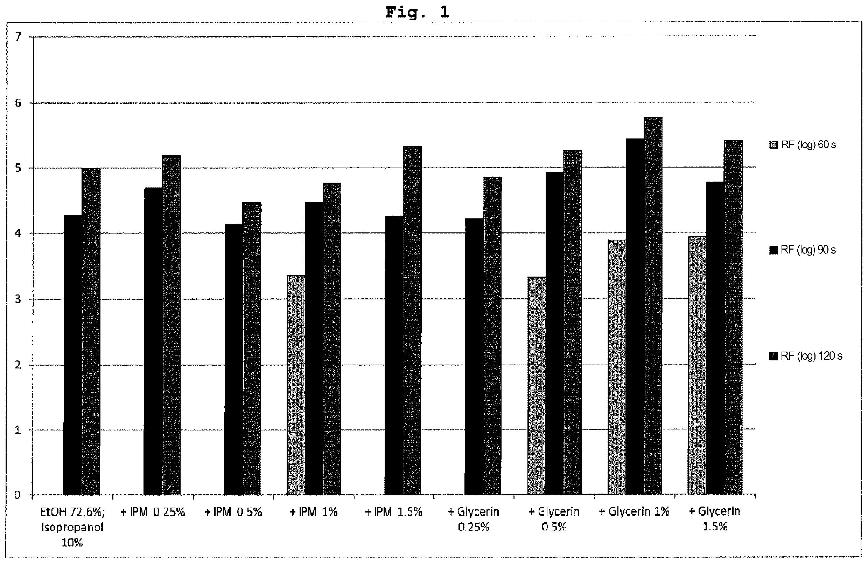

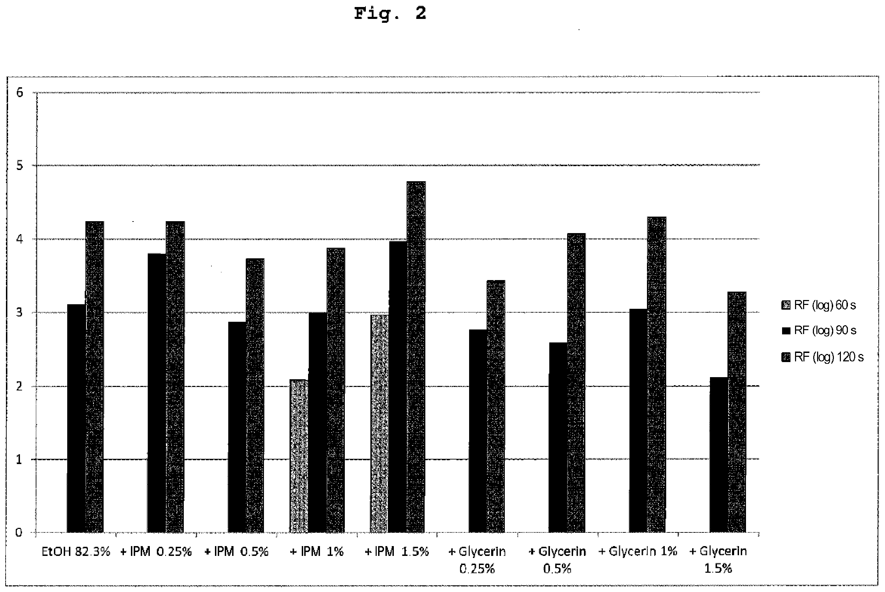

The following two basic formulations were tested in the quantitative suspension test (dirty conditions, *E. coli*)*:*
- mixture of EtOH (72.6 g/100 g) with isopropanol (10 g/100 g), as comparative value without refatting agent and with the concentration of the respective refatting agent given in Fig. 1, (reference example)
- EtOH (82.3 g/100 g), as comparative value without refatting agent and with the concentration of the respective refatting agent given in Fig. 2 (with IPM = according to the invention, with glycerin = comparison).

As can be seen from the examples, glycerin has an effect on the efficacy of alcoholic disinfectants in the quantitative suspension test, mainly in the range of amounts of 1 wt% or more that is of interest with respect to use as refatting agent. The effect whereby glycerin actually even lowers the efficacy in the quantitative suspension test in an amount of 1.5 wt%, is especially pronounced in the case of alcoholic disinfectants that contain over 80 wt% ethanol. In contrast, isopropyl myristate in an amount of 1.5 wt% increases the efficacy of an ethanolic disinfectant in the quantitative suspension test.

The data, especially in Fig. 2, thus show that, surprisingly, with increasing concentration of isopropyl myristate, the efficacy increases (starting from a concentration of ≥ 1 wt% isopropyl myristate, already at 60 s there is a significant reduction of *Escherichia coli* (RF >1 log)). In contrast, addition of glycerin in corresponding concentration does not lead to any improvement in the efficacy of the ethanolic solution. Starting from a concentration of > 1 wt% glycerin, at any rate with a disinfectant without isopropanol, the efficacy is even impaired.

### Example 2

### Haptic assessment

The following products were assessed (Table 1):

**Table 1: Composition of the agents undergoing haptic assessment**

| | |
|---|---|
| A | 90.00 parts by weight ethanol 96%, denatured with MEK, i.e. 83.7 wt% ethanol |
| | 1.40 parts by weight isopropyl myristate |
| | 6.80 parts by weight purified water |
| | 0.80 parts by weight propylene glycol |
| | 0.70 parts by weight cetearyl octanoate |
| | 0.30 parts by weight povidone K |
| B (*) | Commercially available hand disinfectant, composition according to manufacturer's data: 100 g of solution contains 99% ethanol 85.0 g, other constituents: butan-2-one, 1-propanol (Ph. Eur.), tetradecan-1-ol, glycerin 85%, purified water |
| C (*) | Commercially available hand disinfectant, composition according to manufacturer's data: ethanol 45 g, 1-propanol 18 g, other constituents: purified water, diisopropyl adipate, macrogol-6-glycerin caprylocaprate, dexpanthenol, (+/-)-alpha-bisabolol, odour substances (contain limonene and linalool), allantoin. |
| D (*) | Commercially available hand disinfectant, composition according to manufacturer's data: 100 g of solution contains 99% ethanol 95.0 g, other constituents butan-2-one, glycerin, tetradecan-1-ol, benzine |

| | |
|---|---|
| (*) = Comparative Example | |

It was found, surprisingly, that a formulation that contains fatty acid ester (such as isopropyl myristate) instead of glycerin, is assessed in a blinded proband test as at least as pleasant as commercially obtainable glycerin-based products with high ethanol content (see Table 2).

**Table 2: Presentation of the assessment of various ethanolic formulations for disinfection of the hands**

| | Number of assessments (scores) | | | | | | | | |
|---|---|---|---|---|---|---|---|---|---|
| | 1 | 2 | 3 | 4 | 5 | 6 | Average score | n | Ranking |
| A | 2 | 2 | 3 | 3 | | | 2.7 | 10 | 1 |
| B (*) | 1 | 5 | 2 | | 2 | | 2.7 | 10 | 1 |
| C (*) | | 3 | 2 | 3 | 1 | 1 | 3.5 | 10 | 3 |
| D (*) | | 1 | 2 | 2 | 2 | 3 | 4.4 | 10 | 4 |

| | | | | | | | | | |
|---|---|---|---|---|---|---|---|---|---|
| (*) = Comparative Example | | | | | | | | | |

## Claims

1. Alcoholic disinfectant, comprising
a) 81.5 to 92.5wt% ethanol, relative to the weight of the disinfectant, and
b) 0.3 to 2.0 wt% of isopropyl myristate;
wherein the disinfectant
i. comprises less than 0.1 wt% glycerin, relative to the weight of the disinfectant, and
ii. comprises less than 5.0 wt% perfume, relative to the weight of the disinfectant,
the disinfectant comprising 0.05 to 5.0 wt% ingredients other than water and ingredients a) and b).

2. Disinfectant according to one of the preceding claims, **characterized in that** component b) is present in an amount from 0.5 to 1.8 wt%.

3. Disinfectant according to one of the preceding claims, **characterized in that** it is free from water-insoluble polymeric amines.

4. Disinfectant according to one of the preceding claims, **characterized in that** it is free from amino-substituted polystyrenes.

5. Disinfectant according to one of the preceding claims, **characterized in that** it further comprises:
c) 0.5 to 1.5 wt% fatty alcohol,
d) 0.4 to 1.0 wt% diol,
e) 0.05 to 0.15 wt% bispyridinium alkane,
f) 0.3 to 1.0 wt% chlorhexidine derivative,
g) 0.05 to 0.3 wt% glycerin ether,
h) 0.3 to 1.0 wt% fatty acid fatty alcohol ester,
i) 0.4 to 1.0 wt% polyvinylpyrrolidone,
j) 0.3 to 1.0 wt% sugar alcohol,
k) 0.005 to 0.03 wt% of antioxidant,
and/or
l) 0.05 to 0.3 wt% of skin care agent.

## Patentansprüche

1. Alkoholisches Desinfektionsmittel, umfassend
a) 81,5 bis 92,5 Gew.-% Ethanol, bezogen auf das Gewicht des Desinfektionsmittels, und
b) 0,3 bis 2,0 Gew.-% Isopropylmyristat;
wobei das Desinfektionsmittel
i. weniger als 0,1 Gew.-% Glycerin, bezogen auf das Gewicht des Desinfektionsmittels, umfasst und
ii. weniger als 5,0 Gew.-% Parfüm, bezogen auf das Gewicht des Desinfektionsmittels, umfasst,
das Desinfektionsmittel umfassend 0,05 bis 5,0 Gew.-% Bestandteile, die andere als Wasser und Bestandteile a) und b) sind.

2. Desinfektionsmittel nach einem der vorhergehenden Ansprüche, **dadurch gekennzeichnet, dass** Bestandteil b) ist in einer Menge von 0,5 bis 1,8 Gew.-% vorhanden ist.

3. Desinfektionsmittel nach einem der vorhergehenden Ansprüche, **dadurch gekennzeichnet, dass** es frei von wasserunlöslichen polymeren Aminen ist.

4. Desinfektionsmittel nach einem der vorhergehenden Ansprüche, **dadurch gekennzeichnet, dass** es frei von aminosubstituierten Polystyrolen ist.

5. Desinfektionsmittel nach einem der vorhergehenden Ansprüche, **dadurch gekennzeichnet, dass** es ferner umfasst:
c) 0,5 bis 1,5 Gew.-% Fettalkohol,
d) 0,4 bis 1,0 Gew.-% Diol,
e) 0,05 bis 0,15 Gew.-% Bispyridiniumalkan,
f) 0,3 bis 1,0 Gew.-% Chlorhexidinderivat,
g) 0,05 bis 0,3 Gew.-% Glycerinether,
h) 0,3 bis 1,0 Gew.-% Fettsäurefettalkoholester,
i) 0,4 bis 1,0 Gew.-% Polyvinylpyrrolidon,
j) 0,3 bis 1,0 Gew.-% Zuckeralkohol,
k) 0,005 bis 0,03 Gew.-% Antioxidans,
und/oder
l) 0,05 bis 0,3 Gew.-% Hautpflegemittel.

## Revendications

1. Désinfectant à base d'alcool, comprenant
a) de 81,5 à 92,5 % en poids d'éthanol, par rapport au poids du désinfectant, et
b) de 0,3 à 2,0 % en poids de myristate d'isopropyle ;
dans lequel le désinfectant
i. comprend moins de 0,1 % en poids de glycérine, par rapport au poids du désinfectant, et
ii. comprend moins de 5,0 % en poids de parfum, par rapport au poids du désinfectant,
le désinfectant comprenant de 0,05 à 5,0 % en poids d'ingrédients autres que l'eau et les ingrédients a) et b) .

2. Désinfectant selon l'une quelconque des revendications précédentes, **caractérisé en ce que** le composant b) est présent en une quantité de 0,5 à 1,8 % en poids.

3. Désinfectant selon l'une quelconque des revendications précédentes, **caractérisé en ce qu'**il est dépourvu d'aminés polymères insolubles dans l'eau.

4. Désinfectant selon l'une quelconque des revendications précédentes, **caractérisé en ce qu'**il est dépourvu de polystyrènes à substitution amino.

5. Désinfectant selon l'une quelconque des revendications précédentes, **caractérisé en ce qu'**il comprend en outre
c) de 0,5 à 1,5 % en poids d'alcool gras,
d) de 0,4 à 1,0 % en poids de diol,
e) de 0,05 à 0,15 % en poids de bispyridinium alcane,
f) de 0,3 à 1,0 % en poids de dérivé de chlorhexidine,
g) de 0,05 à 0,3 % en poids d'éther de glycérine,
h) de 0,3 à 1,0 % en poids d'ester d'acide gras et d'alcool gras,
i) de 0,4 à 1,0 % en poids de polyvinylpyrrolidone,
j) de 0,3 à 1,0 % en poids d'alcool de sucre,
k) de 0,005 à 0,03 % en poids d'antioxydant,
et/ou
l) de 0,05 à 0,3 % en poids d'agent de soin cutané.
